# EUROPEAN PATENT SPECIFICATION

(11) **EP 1 637 883 B1**
(45) Date of publication and mention of the grant of the patent: **25.11.2009**
(21) Application number: 04745332.9
(22) Date of filing: 26.05.2004
(51) Int. Cl.: G01N 33/543, G01N 33/48

(54) **IMMUNOCHROMATOGRAPHIC METHOD**
IMMUNCHROMATOGRAPHISCHES VERFAHREN
PROCEDE D'IMUNOCHROMATOGRAPHIE

(30) Priority: 27.05.2003 JP 2003149638
(43) Date of publication of application: 22.03.2006
(73) Proprietor: Mitsubishi Kagaku Iatron, Inc., Tokyo 162-0812 (JP)
(72) Inventor: ONO, Tetsuya, MITSUBISHI KAGAKU IATRON, INC., Tokyo 162-0812 (JP); FUJII, Takayuki, MITSUBISHI KAGAKU IATRON, INC., Tokyo 162-0812 (JP); SUGIYAMA, Kazuyuki, MITSUBISHI KAGAKU IATRON, INC., Tokyo 162-0812 (JP); KURODA, Takashi, MITSUBISHI KAGAKU IATRON, INC., Tokyo 162-0812 (JP); KAWAMURA, Masahide, MITSUBISHI KAGAKU IATRON, INC., Tokyo 162-0812 (JP)
(74) Representative: Vossius & Partner
(86) International application number: PCT/JP2004/007180
(87) International publication number: WO 2004/106930

(56) References cited:
- JP-A- 06 011 510
- JP-A- 06 317 596
- JP-A- 58 061 465
- JP-A- 2001 124 772
- SHIFF C J ET AL: "The rapid manual ParaSight-F test. A new diagnostic tool for Plasmodium falciparum infection." TRANSACTIONS OF THE ROYAL SOCIETY OF TROPICAL MEDICINE AND HYGIENE. 1993 NOV-DEC, vol. 87, no. 6, November 1993 (1993-11), pages 646-648, XP002381887 ISSN: 0035-9203

## Description

### TECHNICAL FIELD

The present invention relates to a novel immunochromatographic method.

### BACKGROUND ART

Various products for immunochromatography, in which blood may be used as a sample to be assayed, have been developed, but when whole blood without,a pretreatment is developed on an immnochromatographic membrane or strip, the immnochromatographic development generally does not work well. To avoid this problem, a serum or plasma should be separated from whole blood to remove blood cells or the like. Centrifugation is a major plasma/serum separation method. However, because a centrifuge is required for centrifugation, a general practitioner does not usually perform centrifugation in a consulting room. A method using a filter such as a plasma filter (Fuji film) is known as another method for plasma separation, but it also needs time and is costly. In addition, a large amount of whole blood is necessary because a filtration area of the plasma filter should be widened to separate a sufficient amount of plasma without hemolysis and clogging.

Under these circumstances, some methods in which a pad for a plasma/serum separation is located on an immunochromatographic strip have been developed. More particularly, the separation pad is located under a portion for receiving a whole blood sample in the immunochromatographic strip. When the whole blood sample is applied to the receiving portion, blood cells are maintained in the separation pad so,that they will not move to the immunochromatographic strip in a short time, whereas only serum components are developed on the immunochromatographic strip in advance.

The above methods having such a pad comprise various other devices to prevent erythrocytes in whole blood from hemolyzing and to separate the serum components from blood cells without hemolysis. More particularly, there are many patent applications and registered patents, for example, Japanese Unexamined Patent Publication (Kokai) No. 2002-214236 [patent reference 1] using a carboxymethylcellulose membrane as a membrane for capturing blood cells, Japanese Unexamined Patent Publication (Kokai) No. 2002-350428 [patent reference 2] using propanol and/or acrylamide, or Japanese Patent No. 2940990 [patent reference 3] using a hydrophilic sintered porous substance. The methods disclosed in these patent references are characterized by avoiding hemolysis. This is because hemolysis reddens the whole immnochromatographic developing membrane, including a zone for judgment, and as a result judgment becomes very difficult.

Shiff (Trans R Soc Trop Med Hyg. 1993, 87(6):646-8) discloses the immunochromatographic detection of a marker protein for *Plasmodium falciparum* in the blood of infected patients. Thereby a test called *Para*Sight®-F test is used and the ampholytic detergent Zwittergent® 3-14 is employed for hemolysing the blood prior to immunochromatographic detection.

In fields other than the immunochromatographic method, a hemolytic measuring method [patent reference 4] or a reagent for hemolyzing whole blood with a detergent or the like (Celltac Chemi; Nihon Kohden Corporation) are known. These known assay systems do not have such a problem, because the red pigments do not affect the measurement.
However, in reagents for immunochromatography, in which a developing or coloring agent is used and a visual judgment is made, such as a gold-colloid immunochromatography characterized by staining with red, the staining with red pigments caused by hemolysis is a crucial problem. To avoid hemolysis or a bursting of erythrocytes, blood cells are maintained in the pad for plasma separation under mild conditions, and thus, it takes some time before the immunochromatographic development begins. Further, an amount of plasma developed is affected by the viscosity of a whole blood sample, and thus quantitativeness is often lost. Furthermore, a separation and elution rate of plasma often changes remarkably, dependently on the influence of matrix such as the viscosity of a whole blood sample. Therefore, an apparatus (Roche) in which a color intensity is compensated on the basis of a developing time from the plasma separation by the pad for plasma separation (not from the supply of a whole blood sample) to coloring is available.

Further, it is technically difficult to completely maintain blood cells on the pad for plasma separation for a long time, and some blood cells reach the immunochromatographic developing membrane. In a case, such as an enzyme immunochromatography, in which a second developing solution such as a washing solution or an enzyme-substrate solution should be further developed after the immunochromatographic development of a sample, blood cells that seep out from the pad for plasma separation and reach the immunochromatographic developing membrane inhibit a development of the second solution. As a result, an accurate measured value cannot be obtained by such an immunochromatography.

Furthermore, as a special case, a reagent [patent reference 5] for measuring an antigen on the surface of erythrocytes by immunochromatography is known. In the assay, an immunochromatographic developing membrane having an extremely large pore size (5 to 100 µm) is used, and a developing step on the membrane is carried out without a bursting of erythrocytes. The assay is characterized in that erythrocytes are not burst, as well as the above-mentioned conventional immunochromatographic methods.

Important features of an immunochromatographic method are convenience, rapidity, and low-cost. Such advantages are lost in the above methods using a centrifuge, which needs time and effort, or the use of an expensive prefilter for plasma separation. The problem is overcome in the above methods in which the pad for plasma separation is located on the immunochromatographic strip, but the rapidity or reproducibility thereof should be improved. Further, it is difficult to use the pad in a case (for example, an enzyme immunochromatography) in which another liquid should be developed after developing a sample to be assayed.

[patent reference 1] Japanese Unexamined Patent Publication (Kokai) No. 2002-214236
[patent reference 2] Japanese Unexamined Patent Publication (Kokai) No. 2002-350428
[patent reference 3] Japanese Patent No. 2940990
[patent reference 4] Japanese Unexamined Patent Publication (Kokai) No. 10-221334
[patent reference 5] Japanese Unexamined Patent Publication (Kokai) No. 2000-111555

### DISCLOSURE OF THE INVENTION

### PROBLEMS TO BE SOLVED BY THE INVENTION

As described above, in conventional immunochromatographic methods using blood (i.e., whole blood) as a sample, the whole blood sample is pretreated to perform a serum/plasma separation, or a pad for plasma separation is located on the immunochromatographic strip to delay developing blood cells on the immunochromatographic developing membrane. Both methods are characterized by no bursting of erythrocytes in whole blood and no leaking of red pigments from erythrocytes. However, the serum/plasma preseparation has a disadvantage in cost or effort. The method using the pad for plasma separation on the immunochromatographic strip has a problem with a reproducibility of a measuring time or the like because of the viscosity of whole blood, and is difficult to utilize in an immunochromatography in which another liquid should be developed after developing a sample to be assayed.
An object of the present invention is to provide an immunochromatographic method in which a whole blood sample is pretreated by a rapid and convenient method, a judgment is not affected by a hemolysis of whole blood which causes a temporary coloring of the immunochromatographic developing membrane with red, and the whole blood sample may be analyzed rapidly, conveniently, and at a low cost.

### MEANS FOR SOLVING THE PROBLEMS

The problem may be solved by an immunochromatographic method of the present invention, characterized by comprising the steps of:
(1) preparing a sample derived from whole blood by hemolysis of the whole blood and a treatment for enabling chromatographic development (hereinafter referred to as a step for preparing a sample derived from whole blood or a preparative step),
(2) developing the resulting sample on a developing membrane for immunochromatography (hereinafter referred to as a developing step), and
(3) developing a washing liquid on the developing membrane for immunochromatography to thereby remove an erythrocyte-derived red pigment from the developing membrane for immunochromatography (hereinafter referred to as a step for removing a red pigment or a removing step), wherein
   the sample derived from whole blood is prepared by bringing the whole blood into contact with a nonionic detergent to thereby hemolyze the whole blood and solubilize a component contained in the whole blood and having a particle size larger than a pore size of the developing membrane for immunochromatography.
It is described in the context of the present invention that the treatment for enabling chromatographic development is a treatment for solubilizing, removing, or changing into a small-sized particle a component contained in the whole blood and having a particle size larger than a pore size of the developing membrane for immunochromatography.

It is further described in the context of the present invention that the sample derived from whole blood is prepared by passing the whole blood through a filter to thereby hemolyze the whole blood and remove a component contained in the whole blood and having a particle size larger than a pore size of the developing membrane for immunochromatography.
It is further described in the context of the present invention that the sample derived from whole blood is prepared by disrupting under a high pressure or sonicating the whole blood to thereby hemolyze the whole blood and change into a small-sized particle a component contained in the whole blood and having a particle size larger than a pore size of the developing membrane for immunochromatography.
It is further described in the context of the present invention that the sample derived from whole blood is prepared by bringing the whole blood into contact with an organic solvent to thereby hemolyze the whole blood and solubilize a component contained in the whole blood and having a particle size larger than a pore size of the developing membrane for immunochromatography.
The present invention relates to a kit for immunochromatography characterized by comprising (1) a strip for immunochromatography and (2) a diluting liquid for whole blood containing a nonionic detergent, wherein the diluting liquid contains the nonionic detergent at a concentration such that when whole blood is diluted with the diluting liquid to prepare a sample for immunochromatography, the whole blood is hemolyzed.

### EFFECTS OF THE INVENTION

According to the immunochromatographic method of the present invention, a means for avoiding hemolysis of whole blood, which is essential in conventional methods, is not necessary. Further, a whole blood sample may be immunochromatographically measured at a low cost by a convenient pretreatment of the whole blood sample and immunochromatographic development. Furthermore, the whole blood sample may be measured conveniently and rapidly, with few affects caused by variations in the viscosity of whole blood.

### BEST MODE FOR CARRYING OUT THE INVENTION

The above problem may be resolved by the method of the present invention as described below. That is, whole blood is hemolyzed, for example, by solubilizing or bursting erythrocytes. After one or more components having a particle size larger than a pore size of the developing membrane for immunochromatography, which are contained in the whole blood, are, for example, solubilized, removed, or changed into a small-sized particle, the obtained whole-blood-derived sample is developed. Therefore, clogging of pores in the immunochromatographic developing membrane does not occur during the development. After the development of the whole-blood-derived sample, another liquid is developed on the developing membrane to wash out red components (mainly hemoglobin) generated by hemolysis, on the developing membrane.

A pore size of a developing membrane commonly used in immunochromatography is generally approximately 2 to 20 µm. When blood cells (for example, an erythrocyte having a diameter of approximately 8 µm, a leukocyte having a diameter larger than that of the erythrocyte, or a platelet having a diameter slightly smaller than that of the erythrocyte) or aggregates derived from fibrin or phospholipids are dissolved and solubilized with, a nonionic detergent, the treated sample can be developed on such a developing membrane without a clogging of pores in the developing membrane. The whole-blood-derived sample causes coloring with red of the immunochromatographic developing membrane. The red components derived from hemolyzed whole blood may be removed by washing out the developing membrane with, for example, a washing liquid or a liquid containing a substrate for an enzyme method. After the washing, a stain line for judgment colored by, for example, an immunological or enzymic reaction can be observed. The present invention is based on the above findings.

In the preparative step in the method of the present invention, a method for preparing the whole-blood-derived sample is not particularly limited, so long as whole blood is hemolyzed by using a nonionic detergent and a treatment for enabling chromatographic development may be performed.
The term "treatment for enabling chromatographic development" as used herein means a treatment which enables a chromatographic development on an immunochromatographic developing membrane in the following developing step. More particularly, the treatment means, but is not limited to, a treatment for, for example, solubilizing, removing, or changing into a small-sized particle a component contained in whole blood and having a particle size larger than a pore size (preferably the lower limit of pore sizes) of a developing membrane for immunochromatography [for example, various blood cells (such as an erythrocyte, various leukocytes, or a platelet), membranes derived from hemolyzed blood cells (such as an erythrocyte membrane, various leukocyte membranes, or a platelet membrane), or aggregates derived from fibrin or phospholipids].

As the method for preparing the whole-blood-derived sample as described and disclosed in the context of this invention, there may be mentioned, for example, a method using a detergent, a method using a filter, a method utilizing disruption under a high pressure or sonication, or a method using an organic solvent. In the preparative step, any one of the above methods may be performed alone, or two or more methods may be performed as a combination thereof.

In the method using a detergent, the whole-blood-derived sample is prepared by bringing whole blood taken from a subject into contact with a detergent to thereby hemolyze the whole blood and solubilize one or more components having a particle size larger than a pore size of an immunochromatographic developing membrane contained in the whole blood. In the method using a detergent, components having a particle size larger than a pore size of an immunochromatographic developing membrane contained in the whole blood are solubilized with the detergent. As a result, the whole-blood derived sample can be developed in the following developing step without a clogging of pores in the immunochromatographic developing membrane.

A detergent tends to assemble at an interface between two substances, and changes the properties of the interface. A detergent molecule having such properties is composed of groups having opposite features, i.e., a lipophilic group and a hydrophilic group. A detergent which may be used in the present invention is not particularly limited, so long as it has such properties. As the detergent, there is mentioned, a nonionic detergent.

Nonionic detergents include, for example, polyoxyethylene alkylether, polyoxyethylene alkylallylether, polyoxyethylene derivatives, sorbitan fatty acid ester, polyoxyethylene sorbitan fatty acid ester, polyoxyethylene sorbitol fatty acid ester, glycerin fatty acid ester, polyoxyethylene alkylamine, and alkylalkanolamide.
In the method as described and disclosed in the context of the present invention, for example, any one of such nonionic detergents, but not limited to the above-mentioned nonionic detergents, may be used alone, or two or more detergents may be used as a combination thereof. Further, the detergent(s) may be used together with an additional agent such as salts, urea, and/or an organic solvent, or an additional treatment such as filtration (through a filter), disruption under a high pressure, or sonication.

When the nonionic detergent is used, a concentration thereof may be appropriately selected. An applicable range of concentration depends on each detergent to be used, and is preferably around a critical micelle concentration or more than a critical micelle concentration. A liquid containing the detergent(s) prepared by adjusting a concentration of each detergent is referred to as a detergent liquid. When a detergent concentration in the detergent liquid is too high, the viscosity of the detergent liquid sometimes becomes higher than that of whole blood, depending on properties of the detergent. In the case, it is difficult to mix whole blood with the detergent liquid, and as a result, the whole blood cannot be sufficiently solubilized. In contrast, when a detergent concentration in the detergent liquid is too low, the solubilization of whole blood is sometimes too slow or becomes impossible, and as a result, the immunochromatographic development cannot be normally carried out.

More particularly, when polyoxyethylene monolaurate (Tween 20™; Sigma), a polyoxyethylene sorbitol fatty acid ester classified into a nonionic detergent, is used alone, a final concentration thereof in the reaction of hemolysis is preferably 2 to 30%, more preferably 3 to 20%, most preferably 10 to 20%.
When polyethylene glycol mono-para-iso-octylphenyl ester (Triton X-100™; Sigma), a polyoxyethylene alkylether classified into a nonionic detergent, is used alone, a final concentration thereof in the reaction of hemolysis is preferably 0.1 to 30%, more preferably 0.3 to 20%, most preferably 0.5 to 10%.
When polyoxyethylene laurylether (Emulgen 108™; Kao), a polyoxyethylene alkylether classified into a nonionic detergent, is used alone, a final concentration thereof in the reaction of hemolysis is preferably 0.01 to 20%, more preferably 1 to 20%, most preferably 2.5 to 10%.

Even if a detergent concentration is less than the exemplified concentration ranges, the same effects may be obtained by using plural detergents as a combination thereof. For example, when five kinds of detergents (i.e., Emulgen 108, Triton X-100, Tween 20, Amphitol 86B, and CHAPS) are independently used alone at 0.1% as a final concentration in the reaction of hemolysis, solubilization of whole blood, immunochromatographic staining, and immunochromatographic development cannot be performed, as shown in Example 1. However, when the same detergents are used as a combination thereof (each concentration = 0.1%), solubilization of whole blood, immunochromatographic staining, and immunochromatographic development can be performed, as shown in Example 3.

In contrast, even if a detergent concentration is more than the exemplified concentration range, the same effects may be obtained by using a commercially available detergent diluted to an appropriated concentration. For example, when polyethylene glycol mono-para-iso-octylphenyl ester (Triton X-100) is marketed as "Detergent X (10% aqueous solution)" and is mixed with an equal volume of whole blood, the final concentration of "Detergent X" is 50%, but the substantial concentration of Triton X-100 is 5%, and as a result, the same effects may be obtained.
As a solvent for diluting the detergent, not only a 100% aqueous solution, but also a liquid containing an organic solvent may be used for hemolysis and immunochromatographic development. For example, PB 40 is a 40% solution of paramityl trimethyl ammonium chloride, containing 20 to 30% of isopropanol.
A preferred concentration varies in accordance with, for example, a pretreatment of whole blood, the above exemplified optional methods, and/or properties of an immunochromatographic test, and thus, the detergent concentration is not limited to the above exemplified ranges.

A ratio of the detergent liquid to whole blood is an important factor for a normal immunochromatographic development. When the ratio of the detergent liquid (i.e., detergent content) is too low, components contained in whole blood sometimes are not sufficiently solubilized, and as a result, a normal immunochromatographic development is inhibited. Further, an increased content of the detergent liquid is preferred for a normal and stable immunochromatographic development in a shorter time. In contrast, when the ratio of the detergent liquid is too high, the ratio of whole blood is relatively decreased, and as a result, a sensitivity in the measurement is sometimes lowered. An applicable ratio of the detergent liquid to whole blood depends on each detergent to be used or a concentration thereof, and the ratio (S/WB) of the detergent liquid (S) to whole blood (WB) is preferably 1/10 (vol/vol) or more.

In addition to the above effects, a concentration of the detergent liquid or a ratio thereof to whole blood sometimes affects an inhibition to reactions in immunochromatography or an increased background derived from non-specific staining. Therefore, it is preferred to select optimum conditions (such as a detergent to be used, a concentration thereof, a ratio thereof to whole blood, or a mixing time) in accordance with, for example, a measuring method, an item to be measured, or a measuring time.
A certain detergent sometimes inhibits various binding reactions (such as an antigen-antibody reaction), enzyme reactions, or properties of gold colloid or the like on the immunonochromatography, even if the detergent is used at a concentration capable of immunonochromatographic development. To determine an optimum concentration thereof, it is preferred to independently and carefully examine optimum conditions with respect to each immunochromatography and/or each item to be measured.

In the method using a filter, the whole-blood-derived sample is prepared by passing whole blood taken from a subject through a filter to thereby hemolyze the whole blood and remove one or more components having a particle size larger than a pore size of an immunochromatographic developing membrane contained in the whole blood. In the method using a filter, components having a particle size larger than a pore size of an immunochromatographic developing membrane contained in the whole blood are removed by the filter. As a result, the whole-blood derived sample can be developed in the following developing step without a clogging of pores in the immunochromatographic developing membrane.

As a filter which may be used in the preparative step, there may be mentioned, for example, a syringe filter. After whole blood is collected into an appropriate syringe [for example, 1-mL syringe (Terumo)] via a tube, a syringe filter is attached to the top of the syringe, and the whole blood in the syringe may be filtrated through the syringe filter by pressure. By the pressure during filtration, blood cells are broken, aggregates having a size larger than a pore size of the filter (for example, membrane fragments derived from broken erythrocytes, or aggregates derived from lipid or the like) are removed, and only particles having a size less than a pore size of the filter are collected in a resulting filtrate. A pore size of the filter is preferably less than a pore size (more preferably the lower limit of pore sizes) of a developing membrane for immunochromatography. For example, when an average pore size of an immunochromatographic developing membrane is 5 µm, a pore size of the filter is preferably less than 5 µm. When pore sizes of an immunochromatographic developing membrane are 5 to 8 µm, a pore size of the filter is preferably 5 µm or less. In this connection, the pore size of an immunochromatographic developing membrane means a range containing approximately 80% of all pore sizes, and the pore size of a filter means the maximum pore size.

In the method utilizing disruption under a high pressure or sonication, the whole-blood-derived sample is prepared by disrupting under a high pressure or sonicating whole blood taken from a subject to thereby hemolyze the whole blood and change into a small-sized particle one or more components having a particle size larger than a pore size of an immunochromatographic developing membrane contained in the whole blood. In the method utilizing disruption under a high pressure or sonication, components having a particle size larger than a pore size of an immunochromatographic developing membrane contained in the whole blood are changed into a small-sized particle by disruption under a high pressure or sonication. As a result, the whole-blood derived sample can be developed in the following developing step without a clogging of pores in the immunochromatographic developing membrane.

As the sonication which may be used in the preparative step, there may be mentioned, for example, a supersonic treatment commonly used for cell disruption. As the disruption under a high pressure, there may be mentioned, for example, an apparatus for loading pressure such as a French press. The French press can load a high pressure to a sample containing cells, and can disrupt cells by drastically reducing pressure while collecting a portion of the sample. Therefore, the French press is useful in disrupting and dispersing components having a particle size larger than a pore size of an immunochromatographic developing membrane contained in the whole blood.
The method for preparing the whole-blood-derived sample as described and disclosed in the context of the present invention is not particularly limited, so long as it can hemolyze whole blood, and the above components contained in the whole blood can be solubilized, changed into a small-sized particle, or removed.

An organic solvent which may be used in the preparative step is not particularly limited, so long as it can hemolyze whole blood, and components having a particle size larger than a pore size of an immunochromatographic developing membrane contained in the whole blood can be solubilized. As the organic solvent, there may be mentioned, for example, ketones (such as acetone), alcohols (such as methanol, ethanol, or 2-propanol), or acetonitrile. A concentration of the organic solvent may be appropriately selected in accordance with an organic solvent to be used or the conditions (for example, conditions when mixing with whole blood, or experimental conditions in immunochromatography or measurement), as previously described in the above section of detergents.

The developing step in the method as disclosed in the context of the present invention may be carried out in accordance with a developing step in a conventional immunographic method, except that the whole-blood-derived sample obtained in the preparative step is used. In the whole-blood-derived sample used in the developing step, components which may cause clogging of pores in the immunochromatographic developing membrane are previously solubilized, removed, or changed into a small-sized particle. Therefore, immunochromatographic development can be carried out without a clogging of pores in the immunochromatographic developing membrane.

After the developing step, the immunochromatographic developing membrane is colored with red pigments derived from disrupted erythrocytes in whole blood, and thus it is difficult to observe the staining of a judgment line or the like. Therefore, after the developing step, in the removing step in the method of the present invention, another liquid is developed on the immunochromatographic developing membrane to remove the red pigments from the immunochromatographic developing membrane.
The liquid for removing red pigments (hereinafter referred to as removing liquid) is not particularly limited, so long as it can remove the red pigments by developing it on the immunochromatographic developing membrane. As the liquid for removing red pigments, a washing liquid for simply washing out red pigments (for example, water or a buffer) may be used. In a case (such as an enzyme immunochromatography) in which a substrate for enzymic coloring is developed after the first development, a liquid containing substrate may be used as the removing liquid to wash out the red pigments.

The start of the development of the removing liquid is not particularly limited, so long as the whole-blood-derived sample is sufficiently developed and red pigments can be removed. Generally, after the development of the whole-blood-derived sample begins, the development of the removing liquid may be started. Further, the development of the removing liquid may be started just before the start of the development of the whole-blood-derived sample, by appropriately selecting the starting position of each development.

The kit as disclosed in the context of the present invention for immunochromatography comprises at least (1) a strip for immunochromatography and (2) a diluting liquid for whole blood containing a detergent. As the detergent, a detergent used in the preparative step may be used. A concentration of the detergent contained in the diluting liquid for whole blood is not particularly limited, so long as when whole blood is diluted with it to prepare a sample for immunochromatography (i.e., whole-blood-derived sample), the whole blood may be hemolyzed.
The diluting liquid for whole blood may further contain a substance (such as an antibody or an antigen) which specifically binds to an analyte in a sample (i.e., whole blood) and/or an agent (such as proteins, sugars, or high-molecular compounds) for stabilizing reagents.

### EXAMPLES

The present invention now will be further illustrated by, but is by no means limited to, the following Examples.

### Example 1

In this example, whole blood was hemolyzed and solubilized with various detergents to prepare whole-blood-derived samples, and the resulting whole-blood-derived samples were subjected to an enzyme immunochromatographic method to measure specific IgE, in accordance with the following procedures.

### (1) Preparation of strip for enzyme immunochromatography (1-1) Preparation of allergen-immobilized membrane

A nitrocellulose membrane (HF180; pore size = 5 to 8 µm; Millipore) was cut into a rectangular piece (5 mm x 25 mm). An aqueous solution of extracted mite allergen proteins (1 mg/mL) was applied linearly with a width of 1 mm at the position of 15 mm from an end (upstream) of the membrane. In this connection, the aqueous solution was previously prepared by diluting a Dermatophagoides pteronyssinus extract (Glia) with a 5 mmol/L borate buffer (pH 8.5) and dialyzing the diluted solution.
The membrane was allowed to stand at room temperature for an hour followed by standing at 37°C for 30 minutes to immobilize extracted mite proteins on the membrane. The membrane was kept in a silica gel desiccator at room temperature overnight to obtain a mite allergen-immobilized membrane.

### (1-2) Preparation of anti-IgE antibody labeled with enzyme

An anti-human-IgE mouse monoclonal antibody was digested with pepsin. After the reaction mixture was reduced with 2-mercaptoethylamine, a gel filtration was carried out to obtain a purified Fab' fragment (1 mg).
Meanwhile, bovine intestinal alkaline phosphatase (2 mg) was diluted with a phosphate buffer to a concentration of 2 mg/mL. After dialysis, the diluted solution was mixed with 100 µL of 7.5 mmol/L N-succinimidyl-3-(2-pyridylthio)propionate (SPDP). After the reaction was performed at 4°C for 5 hours, the whole was dialyzed in a phosphate buffer to obtain pyridylthiopropionate (PDP)-bound alkaline phosphatase
After the anti-IgE antibody Fab' (1 mL) was mixed with an equal volume of the PDP-bound alkaline phosphatase (1 mL), 40 µL of 1 mol/L hydroxylamine was further added. After the reaction was performed at 4°C for 3 days, unreacted anti-IgE antibody Fab' and PDP-bound alkaline phosphatase were removed by gel filtration using a gel filtration column (TSKgel™ G3000SW; Toso) to obtain a solution of enzyme-labeled anti-IgE antibody.

### (1-3) Preparation of pad containing enzyme-labeled antibody

After the enzyme-labeled anti-IgE antibody solution prepared in Example 1(1-2) was diluted with 5 mmol/L phosphate buffer (pH 7.2) containing 5.0% sucrose, 10 µL of the diluted solution (1 µg antibody) was sprayed to an absorbent pad (5 mm x 5 mm, PREM1420; Pole). The pad was dried in a silica gel desiccator at room temperature under reduced pressure (not more than 100 mmHg) overnight to obtain an enzyme-labeled antibody pad

### (1-4) Preparation of pad for receiving sample

A glass fiber pad was cut into a rectangular piece (5 mm x 18 mm). The piece was immersed in an aqueous solution containing 0.5% sucrose, 0.2% Tween 20, and 0.1% polyvinyl alcohol (degree of polymerization = approximately 500). After an excess of liquid was removed from the piece, the piece was air-dried to obtain a sample-receiving pad.

### (1-5) Preparation of absorbent pad

A cellulose pad (AP25; Millipore) was cut into a rectangular piece (5 mm x 20 mm) to obtain an absorbent pad.

### (1-6) Preparation of strip for immunochromatography

A plastic adhesive sheet (BioDot) was cut into a rectangular piece (5 mm x 60 mm). The sample-receiving pad [prepared in Example 1(1-4)], the alkaline phosphatase (AP)-labeled antibody pad [prepared in Example 1(1-3)], the mite-allergen-immobilized membrane [prepared in Example 1(1-1)], and the absorbent pad [prepared in Example 1(1-5)] were attached on the adhesive sheet in this order from upstream to downstream with respect to the developing direction to obtain a strip for immunochromatography. In this connection, adjacent pieces attached on the adhesive sheet were overlapped with each other at a width of approximately 1 mm.

### (2) Preparation of whole-blood-derived sample by hemolysis and solubilization and measurement in immunochromatography (2-1) Preparation of detergent liquids

Deionized water, physiological saline (150 mmol/L sodium chloride liquid), and a 10 mmol/L phosphate buffer (pH 7.5, 150 mmol/L sodium chloride) were prepared. Triton X-100 (Sigma), Tween 20 (Sigma), sodium dodecyl sulfate, Emulgen 108 (Kao), PB40 (NOF Corporation), Amphitol 86B (Kao), and CHAPS (Dojindo) were independently diluted with the above phosphate buffer to concentrations of 0.2%, 1.0%, 5.0%, and 20%.

### (2-2) Preparation of samples by mixing equal volumes of whole blood and detergent liquid and observation on hemolysis

Human whole blood (100 µL) collected using a tube containing heparin anticoagulant was mixed with an equal volume of each detergent liquid (100 µL) to prepare each sample. After 10 minutes from the mixing, hemolysis in each sample was observed visually. The "hemolysis" was judged on the basis of a transparent state caused by a solubilization of erythrocytic membrane and elution of red pigments such as hemoglobin from erythrocytes.

### (2-3) Enzyme immunochromatographic measurement

Each aliquot (50 µL) of whole-blood-derived samples prepared by hemolysis and solubilization was dispensed into each well of a microplate. The strip for immunochromatography was stood so that the sample-receiving pad was put into the well, and a chromatographic development of samples was carried out by a capillary phenomenon. After 10 minutes from the beginning of the development, 200 µL of a solution of 5-bromo-4-chloro-3-indolyl-phosphoric acid (BCIP; Boeringer Mannheim) was chromatographically developed.
As the above samples, sample 1 (mite-specific IgE = 0 IU/mL), sample 2 (mite-specific IgE = 3 IU/mL), and sample 3 (mite-specific IgE = 10 IU/mL) were used. The amounts of mite-specific IgE were measured by a conventional quantitative analysis (AlaSTAT specific IgE antibody measuring kit; obtained from Iatron and manufactured by DPC, US) and shown by IU/mL, an international standard unit.

After the chromatographic development, a degree of staining on the allergen-immobilized area (the band having a width of 1 mm) was judged visually, and the results are shown in Tables 1 to 8. The symbols "-", "±", "+", and "++" in Tables 1 to 8 (and Tables 9 to 19 described below) mean "not colored", "slightly colored", "clearly colored", and "strongly colored", respectively. A concentration of each detergent liquid in Tables shows a concentration thereof used in Example 1(2-2), and thus, the final concentration thereof in the reaction of hemolysis was a half of the concentration shown in the Tables

**Table 1 (comparative)**

| Sample prepared by mixing whole blood with equal volume of non-detergent liquid | | | | |
|---|---|---|---|---|
| Conc. of detergent | | Deionized water | Physiological saline | Phosphate buffer |
| Hemolysis | | Hml | Not | Not |
| Mite-specific IgE antibody | | | | |
| | (IU/mL) | | | |
| Sample 1 | 0 | FCD | FCD | FCD |
| Sample 2 | 3 | FCD | FCD | FCD |
| Sample 3 | 10 | FCD | FCD | FCD |

| | | | | |
|---|---|---|---|---|
| [Hml = Hemolyzed; Not = Not hemolyzed; FCD = Failure in chromatographic development] | | | | |

**Table 2**

| Sample prepared by mixing whole blood with equal volume of Tween 20 | | | | | |
|---|---|---|---|---|---|
| Conc. of detergent | | 20% | 5% | 1% | 0.2% |
| Hemolysis | | Hml | Not | Not | Not |
| Mite-specific IgE antibody | | | | | |
| | (IU/mL) | | | | |
| Sample 1 | 0 | - | FCD | FCD | FCD |
| Sample 2 | 3 | + | FCD | FCD | FCD |
| Sample 3 | 10 | ++ | FCD | FCD | FCD |

**Table 3 (comparative)**

| Sample prepared by mixing whole blood with equal volume of sodium dodecyl sulfate | | | | | |
|---|---|---|---|---|---|
| Conc. of detergent | | 20% | 5% | 1% | 0.2% |
| Hemolysis | | Hml | Hml | Hml | Hml |
| Mite-specific IgE antibody | | | | | |
| | (IU/mL) | | | | |
| Sample 1 | 0 | - | - | - | - |
| Sample 2 | 3 | - | - | ± | ± |
| Sample 3 | 10 | - | - | + | + |

**Table 4**

| Sample prepared by mixing whole blood with equal volume of Emulgen 108 | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | - | - | FCD | FCD |
| Sample 2 | 3 | + | + | FCD | FCD |
| Sample 3 | 10 | ++ | ++ | FCD | FCD |

**Table 5**

| Sample prepared by mixing whole blood with equal volume of Triton X-100 | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | - | - | - | FCD |
| Sample 2 | 3 | + | + | + | FCD |
| Sample 3 | 10 | ++ | ++ | ++ | FCD |

**Table 6 (comparative)**

| Sample prepared by mixing whole blood with equal volume of PB40 | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | FCD | - | - | FCD |
| Sample 2 | 3 | FCD | + | + | FCD |
| Sample 3 | 10 | FCD | ++ | ++ | FCD |

**Table 7 (comparative)**

| Sample prepared by mixing whole blood with equal volume of Amphitol 86B | | | | | |
|---|---|---|---|---|---|
| Conc. of detergent | | 20% | 5%. | 1% | 0.2% |
| Hemolysis | | Hml | Hml | Hml | Not |
| Mite-specific IgE antibody | | | | | |
| | (IU/mL) | | | | |
| Sample 1 | 0 | - | - | - | FCD |
| Sample 2 | 3 | + | + | + | FCD |
| Sample 3 | 10 | ++ | ++ | ++ | FCD |

**Table 8 (comparative)**

| Sample prepared by mixing whole blood with equal volume of CHAPS | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | - | - | - | FCD |
| Sample 2 | 3 | + | + | + | FCD |
| Sample 3 | 10 | ++ | ++ | ++ | FCD |

### Example 2

In this example, whole blood was hemolyzed and solubilized with various detergents to prepare whole-blood-derived samples, and specific IgE was measured by a gold colloid immunochromatographic method, in accordance with the following procedures.

### (1) Preparation of strip for gold colloid immunochromatography

### (1-1) Preparation of allergen-immobilized membrane

In accordance with the procedure shown in Example 1(1-1), a mite allergen-immobilized membrane was prepared by spraying an aqueous solution of extracted mite allergen proteins on a nitrocellulose membrane linearly with a width of 1 mm.

### (1-2) Preparation of anti-IgE antibody labeled with gold colloid

An anti-human-IgE mouse monoclonal antibody (1 mg) was diluted with a phosphate buffer (2 mmol/L, pH 7.0) to a concentration of 0.1 mg/mL, and the diluted solution was dialyzed. While stirring 100 mL of a gold colloid suspension (GOLD COLLOID 20; British BioCell International), 10 mL of the aqueous solution of anti-human-IgE mouse monoclonal antibody was added dropwise to the suspension. After stirring at room temperature for 10 minutes, 10 mL of 10% bovine serum albumin (BSA, A-7888; Sigma) was further added dropwise while stirring. After stirring at room temperature for 10 minutes, 15 mL of an aqueous solution containing 5% sucrose and 0.2% Tween 20 was further added. The whole was mixed and centrifuged 16,000×g for an hour at 10°C to remove a supernatant.

The remaining precipitated antibody labeled with gold colloid was resuspended in 100 mL of a borax buffer (pH 8.0) containing 0.5% sucrose and 0.02% Tween 20. The whole was centrifuged 16,000xg for an hour at 10°C to remove a supernatant. The precipitated antibody labeled with gold colloid was resuspended in 100 mL of the borax buffer (pH 8.0) containing 0.5% sucrose and 0.02% Tween 20, and the whole was centrifuged 16,000xg for an hour at 10°C to remove a supernatant. The precipitated antibody labeled with gold colloid was resuspended in the borax buffer (pH 8.0) containing 0.5% sucrose and 0.02% Tween 20 so that a particle density became approximately 10¹³ /mL (Absorbance at a wavelength of 520 nm = approximately 14) to obtain a suspension of the anti-IgE antibody labeled with gold colloid.

### (1-3) Preparation of pad containing antibody labeled with gold colloid

The procedure described in Example 1(1-3) was repeated, except that the suspension, prepared in Example 2(1-2), of the anti-IgE antibody labeled with gold colloid was used, to obtain a pad containing the gold-colloid-labeled antibody.

### (1-4) Preparation of strip for immunochromatography

The procedure described in Example 1(1-6) was repeated, except that the pad, prepared in Example 2(1-2), containing the gold-colloid-labeled antibody was used, to obtain a strip for gold colloid immunochromatography.

### (2) Preparation of whole-blood-derived sample by hemolysis and solubilization and measurement in immunochromatography

### (2-1) Preparation of detergent liquids, mixing with whole blood, and observation on hemolysis

In accordance with the procedure shown in Example 1(2-1), various solutions were prepared. In accordance with the procedure shown in Example 1(2-2), human whole blood (100 µL) was mixed with an equal volume of each solution (100 µL) to prepare each sample, and hemolysis in each sample was observed.

### (2-2) Gold colloid immunochromatographic measurement

The procedure described in Example 1(2-3) was repeated, except that the strip for enzyme immunochromatography was replaced with the strip for gold colloid immunochromatography prepared in Example 2(1-4), and the solution of 5-bromo-4-chloro-3-indolyl-phosphoric acid (BCIP; Boeringer Mannheim) was replaced with a phosphate buffer, to perform a gold colloid immunochromatographic method.

After the chromatographic development, a degree of coloring on the allergen-immobilized area (the band having a width of 1 mm) was judged visually, and the results are shown in Tables 9 to 16.

**Table 9 (comparative)**

| Sample prepared by mixing whole blood with equal volume of non-detergent liquid | | | | |
|---|---|---|---|---|
| Conc. of detergent | | Deionized water | Physiological saline | Phosphate buffer |
| Hemolysis | | Hml | Not | Not |
| Mite-specific IgE antibody | | | | |
| | (IU/mL) | | | |
| Sample 1 | 0 | FCD | FCD | FCD |
| Sample 2 | 3 | FCD | FCD | FCD |
| Sample 3 | 10 | FCD | FCD | FCD |

**Table 10**

| Sample prepared by mixing whole blood with equal volume of Tween20 | | | | | |
|---|---|---|---|---|---|
| Conc. of detergent | | 20% | 5% | 1% | 0.2% |
| Hemolysis | | Hml | Not | Not | Not |
| Mite-specific IgE antibody | | | | | |
| | (IU/mL) | | | | |
| Sample 1 | 0 | - | FCD | FCD | FCD |
| Sample 2 | 3 | ± | FCD | FCD | FCD |
| Sample 3 | 10 | + | FCD | FCD | FCD |

**Table 11 (comparative)**

| Sample prepared by mixing whole blood with equal volume of sodium dodecyl sulfate | | | | | |
|---|---|---|---|---|---|
| Conc. of detergent | | 20% | 5% | 1% | 0.2% |
| Hemolysis | | Hml | Hml | Hml | Hml |
| Mite-specific IgE antibody | | | | | |
| | (IU/mL) | | | | |
| Sample 1 | 0 | - | - | - | - |
| Sample 2 | 3 | - | ± | ± | ± |
| Sample 3 | 10 | - | ± | ± | ± |

**Table 12**

| Sample prepared by mixing whole blood with equal volume of Emulgen 108 | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | - | - | FCD | FCD |
| Sample 2 | 3 | + | ± | FCD | FCD |
| Sample 3 | 10 | + | + | FCD | FCD |

**Table 13**

| Sample prepared by mixing whole blood with equal volume of Triton X-100 | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | - | - | - | FCD |
| Sample 2 | 3 | ± | ± | ± | FCD |
| Sample 3 | 10 | + | + | + | FCD |

**Table 14 (comparative)**

| Sample prepared by mixing whole blood with equal volume of PB40 | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | FCD | - | - | FCD |
| Sample 2 | 3 | FCD | ± | ± | FCD |
| Sample 3 | 10 | FCD | + | + | FCD |

**Table 15 (comparative)**

| Sample prepared by mixing whole blood with equal volume of Amphitol 86B | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | - | - | - | FCD |
| Sample 2 | 3 | ± | ± | ± | FCD |
| Sample 3 | 10 | + | + | + | FCD |

**Table 16 (comparative)**

| Sample prepared by mixing whole blood with equal volume of CHAPS | | | | | |
|---|---|---|---|---|---|
| Conc. of detergent | | 20% | 5% | 1% | 0.2% |
| Hemolysis | | Hml | Hml | Hml | Not |
| Mite-specific IgE antibody | | | | | |
| | (IU/mL) | | | | |
| Sample 1 | 0 | - | - | - | FCD |
| Sample 2 | 3 | ± | ± | ± | FCD |
| Sample 3 | 10 | + | + | + | FCD |

### Example 3

In this example, whole blood was hemolyzed and solubilized with mixed liquids containing five detergents at the same concentration to prepare whole-blood-derived samples, and specific IgE was measured by an enzyme immunochromatographic method, in accordance with the following procedures.

### (1) Preparation of strip for enzyme immunochromatography

The procedure described in Example 1(1) was repeated to prepare a strip for enzyme immunochromatography.

### (2) Preparation of mixture liquid of detergents, mixing with whole blood, and observation on hemolysis

Four kinds of mixed liquids each containing five detergents (Triton X-100, Tween 20, Emulgen 108, Amphitol 86B, and CHAPS) at the same concentration (concentration = 0.04, 0.2, 1, or 4%) were prepared using 10 mmol/L phosphate buffer (pH 7.5, 150 mmol/L sodium chloride). Total concentrations of five detergents in the four mixed liquids were 0.2, 1.0, 5.0, and 20%, respectively. In accordance with the procedure shown in Example 1(2-2), human whole blood (100 µL) was mixed with an equal volume of each mixed liquid of detergents (100 µL) to prepare each sample, and hemolysis in each sample was observed.

### (3) Enzyme immunochromatographic measurement

In accordance with the procedure shown in Example 1(2-3), an immunochromatographic measurement and a visual judgment were carried out. The result is shown in Table 17. Each concentration shown in the Table means a concentration of each detergent contained in each mixed liquid used in Example 3(2).

**Table 17**

| Sample prepared by mixing whole blood with equal volume of mixed liquid of plural detergents | | | | | |
|---|---|---|---|---|---|
| Conc. of detergent | | 4% | 1% | 0.2% | 0.04% |
| Hemolysis | | Hml | Hml | Hml | Not |
| Mite-specific IgE antibody | | | | | |
| | (IU/mL) | | | | |
| Sample 1 | 0 | - | - | - | FCD |
| Sample 2 | 3 | + | + | + | FCD |
| Sample 3 | 10 | ++ | ++ | ++ | FCD |

### Example 4 (comparative)

In this example, a syringe filter was used to hemolyze whole blood and remove components contained in the whole blood having a size larger than a pore size of the filter, and the obtained whole-blood-derived sample was used to measure specific IgE by an enzyme immunochromatography.

### (1) Preparation of strip for enzyme immunochromatography

The procedure described in Example 1(1) was repeated to prepare a strip for immunochromatography [nitrocellulose membrane = HF180 (pore size = 5 to 8 µm)]. The same procedure was repeated, except that HF180 was replaced with HF240 (pore size = 3 to 5 µm), to prepare another strip for immunochromatography. Each pore size means a range containing approximately 80% of all pore sizes.

### (2) Preparation of whole-blood-derived sample by treatment with filter and immunochromatographic measurement

### (2-1) Preparation of whole-blood-derived filtrates by treatment with various syringe filters

Human whole blood collected using a tube containing an anticoagulant (heparin) was filtered through a 5 µm syringe filter (Sartorius), a 3 µm syringe filter (Advantec), a 1.2 µm syringe filter (Sartorius), a 0.8 µm syringe filter (Advantec), or a 0.2 µm syringe filter (Advantec) to collect each filtrate. In this connection, a diameter of each syringe filter was 26 mm. After filtration, hemolysis was visually observed. The pore size of each filter means the maximum pore size.

### (2-2) Enzyme immunochromatographic measurement

Two strips for enzyme immunochromatography prepared in Example 4(1) were used to develop each filtrate by immunochromatography in accordance with the method described in Example 1(2-3).
After the development, 200 µL of a solution of 5-bromo-4-chloro-3-indolyl-phosphoric acid (BCIP; Boeringer Mannheim) was chromatographically developed in accordance with the method described in Example 1(2-3). In this connection, the same samples as those described in Example 1(2-3) were used.
After the chromatographic development, a degree of coloring on the allergen-immobilized area (the band having a width of 1 mm) was judged visually, and the results are shown in Tables 18 and 19.

**Table 18**

| Strip (HF180) | | | | | | | |
|---|---|---|---|---|---|---|---|
| Filtrate sample through syringe filter | | | | | | | |
| Pore size of filer (µm) | | Not filtered | 5 | 3 | 1.2 | 0.8 | 0.2 |
| Hemolysis | | Not | Hml | Hml | Hml | Hml | Hml |
| Mite-specific IgE antibody | | | | | | | |
| | (IU/mL) | | | | | | |
| Sample 1 | 0 | FCD | - | - | - | - | - |
| Sample 2 | 3 | FCD | + | + | + | + | + |
| Sample 3 | 10 | FCD | ++ | ++ | ++ | ++ | ++ |

**Table 19**

| Strip (HF240) | | | | | | | |
|---|---|---|---|---|---|---|---|
| Filtrate sample through syringe filter | | | | | | | |
| Pore size of filer (µm) | | not filtered | 5 | 3 | 1.2 | 0.8 | 0.2 |
| Hemolysis | | Not | Hml | Hml | Hml | Hml | Hml |
| Mite-specific IgE antibody | | | | | | | |
| | (IU/mL) | | | | | | |
| Sample 1 | 0 | | FCD | FCD | - | - | - |
| Sample 2 | 3 | FCD | FCD | + | + | + | + |
| Sample 3 | 10 | FCD | FCD | ++ | ++ | ++ | ++ |

### INDUSTRIAL APPLICABILITY

The immunochromatographic method of the present invention may be applied to the analysis of a whole blood sample.

## Claims

1. An immunochromatographic method **characterized by** comprising the steps of:
(1) preparing a sample derived from whole blood by hemolysis of the whole blood and a treatment for enabling chromatographic development,
(2) developing the resulting sample on a developing membrane for immunochromatography, and
(3) developing a washing liquid on the developing membrane for immunochromatography to thereby remove an erythrocyte-derived red pigment from the developing membrane for immunochromatography,
wherein the sample derived from whole blood is prepared by bringing the whole blood into contact with a nonionic detergent to thereby hemolyze the whole blood and solubilize a component contained in the whole blood, said component having a particle size larger than a pore size of the developing membrane for immunochromatography.

2. A kit for immunochromatography **characterized by** comprising
(1) a strip for immunochromatography and
(2) a diluting liquid for whole blood containing a nonionic detergent,
wherein the diluting liquid contains the nonionic detergent at a concentration such that when whole blood is diluted with the diluting liquid to prepare a sample for immunochromatography, the whole blood is hemolyzed.

## Patentansprüche

1. Immunchromatographisches Verfahren, **dadurch gekennzeichnet, dass** es die Schritte umfasst:
(1) das Zubereiten einer Probe, die aus Vollblut stammt, durch Hämolyse des Vollbluts und eine Behandlung, um eine chromatographische Entwicklung zu ermöglichen,
(2) das Entwickeln der sich ergebenden Probe auf einer Entwicklungsmembran für Immunchromatographie, und
(3) das Entwickeln einer Waschflüssigkeit auf der Entwicklungsmembran für Immunchromatographie, um dabei ein von Erythrocyten stammendes rotes Pigment von der Entwicklungsmembran für Immunchromatographie zu entfernen,
wobei die Probe, die aus Vollblut stammt, zubereitet wird, indem das Vollblut mit einem nicht-ionischen Detergens in Kontakt gebracht wird, um **dadurch** das Vollblut zu hämolysieren und einen Bestandteil, der in dem Vollblut enthalten ist, zu solubilisieren, wobei der Bestandteil eine Partikelgröße aufweist, die größer ist als eine Porengröße der Entwicklungsmembran für Immunchromatographie.

2. Kit für Immunchromatographie, **dadurch gekennzeichnet, dass** er umfasst:
(1) einen Streifen für Immunchromatographie und
(2) eine Verdünnungsflüssigkeit für Vollblut, die ein nicht-ionisches Detergens enthält,
wobei die Verdünnungsflüssigkeit das nicht-ionische Detergens in einer Konzentration enthält, so dass, wenn das Vollblut mit der Verdünnungsflüssigkeit verdünnt wird, um eine Probe für Immunochromatographie zuzubereiten, das Vollblut hämolysiert wird.

## Revendications

1. Méthode immunochromatographique **caractérisée en ce qu'**elle comprend les étapes de :
(1) préparation d'un échantillon dérivé du sang total par hémolyse du sang total et un traitement permettant le développement chromatographique,
(2) développement de l'échantillon résultant sur une membrane de développement pour immunochromatographie, et
(3) développement d'un liquide de lavage sur la membrane de développement pour immunochromatographie afin d'éliminer le pigment rouge provenant d'érythrocytes de la membrane de développement pour immunochromatographie,
dans laquelle l'échantillon dérivé du sang total est préparé par mise en contact du sang total avec un détergent non ionique afin d'hémolyser le sang total et de solubiliser un composé du sang total, ledit composé ayant une taille particulaire plus grande que la taille des pores de la membrane de développement pour immunochromatographie.

2. Kit immunochromatographique **caractérisé en ce qu'**il comprend
(1) une bande pour immunochromatographie et
(2) un liquide de dilution pour sang total contenant un détergent non ionique,
dans lequel le liquide de dilution contient le détergent non ionique à une concentration telle que, quand le sang total est dilué avec le liquide de dilution pour préparer un échantillon pour immunochromatographie, le sang total est hémolysé.
